# EUROPEAN PATENT APPLICATION

(11) **EP 0 764 445 A2**
(43) Date of publication of application: **26.03.1997**
(21) Application number: 96110147.4
(22) Date of filing: 27.09.1991
(51) Int. Cl.: A61K 39/10, C07K 14/235

(54) **Acellular vaccine**

(30) Priority: 27.09.1990 GB 9021004
(62) Divisional of application: 91917103.3
(71) Applicant: MEDEVA HOLDINGS B.V., 1078 ED Amsterdam (NL)
(72) Inventor: Charles, Ian George, Court Road, London, W1P 9LN (GB); Fairweather, Neil Fraser, Beckenham, Kent BR3 3BS (GB)
(74) Representative: Woods, Geoffrey Corlett

(57) **Abstract**

A vaccine suitable for use in inducing immunity to whooping cough in humans comprises a synergistic combination of the 69kDa antigen of Bordetalla pertussis and toxoided lymphocytosis promoting factor of Bordetalla pertussis.

## Description

The present invention relates to an acellular Bordetella pertussis vaccine and to its use in medicine.

B.pertussis causes a serious and debilitating disease in humans, especially children, which has been kept under control in the developed countries by large scale immunisation programmes. Generally, immunisation has been carried out using a whole cell vaccine derived from cell cultures of B.pertussis and has been found to be relatively effective in preventing the disease. However, concern over adverse reactions, such as fever, local reactions and persistent screaming, has led in recent years to a reduced acceptance of the vaccine and a debate about its continued use.

With the low incidence of the disease in developed countries, the benefit/risk ratio associated with the use of whole cell vaccines is poorly defined. Many clinicians believe however that the risk of adverse reaction outweighs the benefit of immunity to infection. As a result, many children today have not been immunised thus increasing the risk of a whooping cough pandemic. Indeed, in recent years the incidence of whooping cough and resulting infant morbidity have increased as the use of the whole cell vaccine has decreased. Considerable research effort has, therefore, been directed towards the development of an improved vaccine which lacks the components responsible for the adverse effects of the whole cell vaccines, whilst retaining the components necessary to confer protection against the disease.

The search for a safe and effective vaccine has been hampered by the paucity of information regarding the identity and mechanisms of action of the pathogenic, toxic and protective moieties of the bacterial organism contained in whole cell vaccines. Work has, therefore, focused on the isolation and characterisation of the twenty or more surface antigens of the organism and on their efficacy in inducing an immune reaction (J.Am.Med.Soc., 1982, 248(1) 22-23). Examples of antigens that have been investigated include lymphocytosis promoting factor (LPF, otherwise known as pertussis toxin (PT), filamentous haemagglutinin (FHA), lipopolysaccharide (LPS), agglutinogens, dermonecrotic toxin (DNT), heat labile and heat stable toxins, polymorphonuclear leukocyte inhibitor factor, adenylate cyclase, the outer membrane 69kDa antigen (P.69) and other surface components. None of these antigens on their own appears able to provide the basis of an effective vaccine.

The present inventors have found that a combination of the 69kDa antigen and toxoided LPF is surprisingly more potent than the aggregate effect of the individual components. Moreover, the use of a synergistic combination of the 69kDa antigen and toxoided LPF provides a dose-dependent linear response which is parallel to that obtained with a whole cell vaccine (Seagroat, V. and Sheffield, F., J.Biological Standard, 1981, 9, 351-365). The synergistic combination therefore affords the basis of an effective vaccine for use in the prevention of whooping cough.

Accordingly the present invention provides a vaccine containing a synergistic combination of the 69kDa antigen of B.pertussis and toxoided LPF of B.pertussis.

The 69kDa antigen is an N-terminal fragment of the precursor protein, P93, which is processed in vivo by removal of the signal peptide and a C-terminal fragment. It has been previously characterised as an outer membrane protein of B.pertussis. In particular, it has a relative molecular weight of 67 to 73kDa, as determined by 12% (w/w) polyacrylamide gel electrophoresis, and a ratio of proline to glutamic acid of substantially 1:1, as determined by amino acid analysis (EP-A 162639). The amino acid sequence has also been deduced from the cloned DNA sequence (Charles et al, P.N.A.S., 1989, 86, 3554-3558) although, in use with the present invention, this may be varied providing the resulting sequence is at least 90%, and preferably 95%, homologous and has substantially similar biological properties in kind.

The 69kDa antigen may be isolated from cultures of B. pertussis by conventional methods, for example as described by Novotny et al (1985) Infection and Immunity 50, 199-206 and European Publication No. 0162639. Alternatively, it may be obtained using recombinant DNA technology. In this regard, the DNA encoding the 69kDa antigen may be cloned, for example as described by Makoff et al (Bio/Technology, 8, 1990, 1030-1033), and expressed in an appropriate host. It is particularly preferred that bacteria, such as E. coli, or yeast, such as Pichia pastoris, are used as the host. The 69kDa antigen obtained in this way may be insoluble and thus may need to be refolded following the use of guanidinium hydrochloride as denaturant in conventional manner and in any event is preferably purified to a level consistent with its use in a human vaccine.

LPF is a protein of 105 kDa that is released into the extracellular medium by virulent B. pertussis. (Tamura, M et al, 1982, Biochemistry 21, 5516-5522). The DNA sequence of the operon encoding the five subunits has been elucidated (Nicosia et al., 1986 Proc. Natl. Acad. Sci. USA 83, 4631-4635; Iocht & Keith, 1986, Science, 232, 1258-1264).

LPF may be obtained from cultures of B. pertussis following procedures described in the literature, for example the procedure described by Imaizumi et al (Infect. Immun., 1983, 41, 1138-1143). It may be purified using column chromatography or other knob purification procedures. It may be toxoided using a variety of different inactivating agents, such as formaldehyde (formalin), glutaraldehyde, hydrogen peroxide (Sato et al, Lancet, 1984, (1), 122-126) or a combination of any of these reagents.

In addition to chemical detoxification pertussis toxin may be inactivated by genetic manipulation. Pizza et al (1989) Science 246, 497-500, describe a number of genetically engineered alleles of the pertussis toxin gene made by replacing 1 or 2 key amino acids within the enzymatically active S1 subunit.

`The ratio of the 69kDa antigen to toxoided LPF may vary between broad limits, for example from 1:10 to 10:1, but is preferably approximately 1:1 and in any event is such as to achieve a synergistic effect in vaccine potency. The ratio is by weight.

The vaccine of the present invention may optionally contain additional antigens of B.pertussis or other bacteria, such as tetanus and diphtheria.

The vaccine of the present invention is normally associated with a pharmaceutically acceptable vehicle which allows the synergistic combination to be administered to the patient. Administration is usually carried out via the oral or preferably parenteral route. In the case of the parenteral route, the vehicle is generally liquid and the synergistic combination is generally dissolved or suspended in it. An example of a liquid vehicle is physiological saline solution.

The vaccine may also contain an adjuvant for stimulating the immune response and thereby further enhancing the potency of the synergistic combination. Convenient adjuvants for use in the present invention include for example, aluminium hydroxide and aluminium phosphate.

Conveniently the vaccine normally contains a final concentration of antigenic protein in the range of from 0.01 to 5mg/ml, preferably 0.03 to 2 mg/ml, most preferably 0.3 mg/ml. After formulation the vaccine may be incorporated into a sterile container which is then sealed and stored at a low temperature, for example 4^{o}C, or is freeze-dried.

The present invention also provides a method for inducing immunity to whooping cough in humans, comprising the administration to the patient of an effective amount of the vaccine of the present invention.

In order to induce immunity to whooping cough in humans one or more doses of the vaccine are normally administered. Each dose of the vaccine is 0.1 to 2 ml preferably 0.2 to 1 ml, most preferably 0.5 ml.

The present invention will now be exemplified further by reference to the accompanying Figures and the

### Examples.

### Figure Legends

### Figure 1

### Construction of plasmids pPERtac 1, 2, 3, 4, 7 and 8

1a. The DNA and amino acid sequences are SEQ ID NOS:1 and 2 respectively. Plasmid pPERtac2 made by cloning an AvaI - ClaI 1491bp fragment from pMLU6 into pPERtac1. Plasmid pPERtac3 made by cloning a SphI - SacII 851bp fragment from pPERtac4 into pPERtac2. Plasmid pPERtac4 made by cloning an AvaI - DraIII 1875bp fragment from pMLU6 into pPERtac1.
1b. Oligonucleotides 1-4 (SEQ ID NOS:3, 5, 6 and 8 respectively) were cloned between ApaI and BamHI sites of pTETtac2 to form intermediate plasmid pPERtac1. The amino acid sequences shown above oligonucleotides 1 and 3 respectively are SEQ ID NOS:4 and 7.
1c. Oligonucleotide 5 (SEQ ID NO:9) was cloned between BstXI and BamHI of pPERtac 4 to form pPERtac7.
1d. Oligonucleotides 6 and 7 (SEQ ID NOS:10 and 12) were cloned between BglI and BamHI sites of pPERtac7 to form pPERtac8. The amino acid sequence shown above oligonucleotide 6 is SEQ ID NO:11.

### Figure 2

a. Oligonucleotides 8, 9 and 10 are SEQ ID NOS:13, 16 and 14 respectively in which R denotes G or A and Y denotes C or T. Oligonucleotide 9 annealed to oligos 8 and 10, and then cloned between the BglII and EcoRI sites of pPERtac8 provided transformants from which the expression plasmid pPERtac36 was isolated. The amino acid sequence above oligonucleotide 10 is SEQ ID NO:15.
b. Top strand of above oligos after repair in vivo, shown as SEQ ID NO:17 in which R denotes G or A and Y denotes C or T.
c. The changes in (b) present in pPERtac8.
d. The changes in (b) present in pPERtac36,

### Figure 3

SDS polyacrylamide gel of extracts of an induced culture of pPERtac36.

### Figure 4

Construction of the vector pPIC3-605K. Plasmid pPIC1 formed by cloning of synthetic oligonucleotides (upper strand-SEQ ID NO:18; lower strand-SEQ ID NO:19) between AsuII and EcoRI of pA0804.
Plasmid pPIC2 formed from pPIC1.
Adaptor oligonucleotides (upper strand-SEQ ID NO:20, lower strand-SEQ ID NO:21) used to insert a 1.8 kb EcoRI-NheI fragment of pPERtac8 into BamHI - SpeI cut pPIC2 to form the expression vector pPIC3-60.5k.

### Figure 5

A plasmid map of the vector pPIC3-60.5K is shown in part A. Part B gives details of the DNA sequence of the P.69 gene that was used in the construction of pPIC3-60.5K. The DNA fragment used contains the region from AvaI (nt.315) to BglII (nt. 1979) from the 93K precursor gene (Charles et al., 1989, PNAS 86, 3554-3558). This region is flanked by the sequences shown which are derived from the vector pPERtac8 (Makoff et al., Bio/Technology 8, 1990, 1030-1033). The sequences flanking the p69 gene are (left side) SEQ ID NO:22 and (right side) SEQ ID NO:23.

### Figure 6

DNA a dot blot screen of GS115/pPIC3-60.5K Mut^{s} transformants. The filter shown had 56 transformants, a positive control (position E10: multi-copy transformant 'clone 22', identified in a previous screen), and a negative control (E11: GS115). Most of the transformants in the screen gave a similar weak signal, a very small proportion gave a much stronger signal indicative of multi-copy integration (A3, A4, B6: designated nos. 3, 4, and 18). A further four filters were screened in the same way, but no more multi-copy transformants were found.

### Figure 7

Southern blot antigens of pPIC-60.5K transformants probed with H154 DNA. Track 1-BglII-cut pPIC-60.5K; track 2-95115 DNA; tracks 3 and 8 - single-copy transplacements; tracks 4 to 7 - multi-copy clones nos. 3, 4, 18 and 22.

### Figure 8

Western blot of proteins (30 micrograms) from an induced single-copy transformant (track 5) and multi-copy transformants (tracks 6 to 9 - clones 3, 4, 18 and 22, respectively), Tracks 1 to 3 contain pure B.pertussis P.69 in amounts equivalent to 1, 2 and 5% of total cell protein (0.3, 0.6, and 1.5 micrograms), and track 4 contains size markers (not visible).

### Figure 9

Western blot of fermenter samples taken at different times after induction of clone 22 (tracks 1 to 12: -1, 0, 2, 4, 6.75, 22.75, 24.75, 27.75, 30.25, 49.25, 57.25, 71.25 hrs, respectively). Track 13 contains an equivalent loading, 50 micrograms, of protein from a shake flask induction. Tracks 14 to 18 contain pure B.pertussis P.69 equivalent to 0.5, 1, 2, 4, and 8% expression levels.

### Figure 10

Coomassie blue-stained gel of solubilised P.69 preparation (tracks 1 to 3: 2, 5 and 10 microlitres) compared to pure B.pertussis P.69.

### Examples

General information pertinent to the examples which follow :

### Media

- YPD, 1 litre: : 10g yeast extract, 20g peptone, 20g glucose.
- YNBBG, 1 litre: : 13.4g yeast nitrogen base w/o amino acids (Difco), 0.4mg biotin, 20ml glycerol.
- YNBBGCas: : same as YNBBG plus 10g casamino acids.
- YNBBD: : same as YNBBG but 20g glucose instead of 20ml glycerol.
- YNBBM: : same as YNBBG but 5ml methanol instead of 20ml glycerol. YNBBDCas and YNBBMCas had 10g casamino acids per litre.
- Solid media: : plus 20g of agar per litre.

### Example 1: Preparation of 69kDa Antigen of B.pertussis

### Production in E.coli

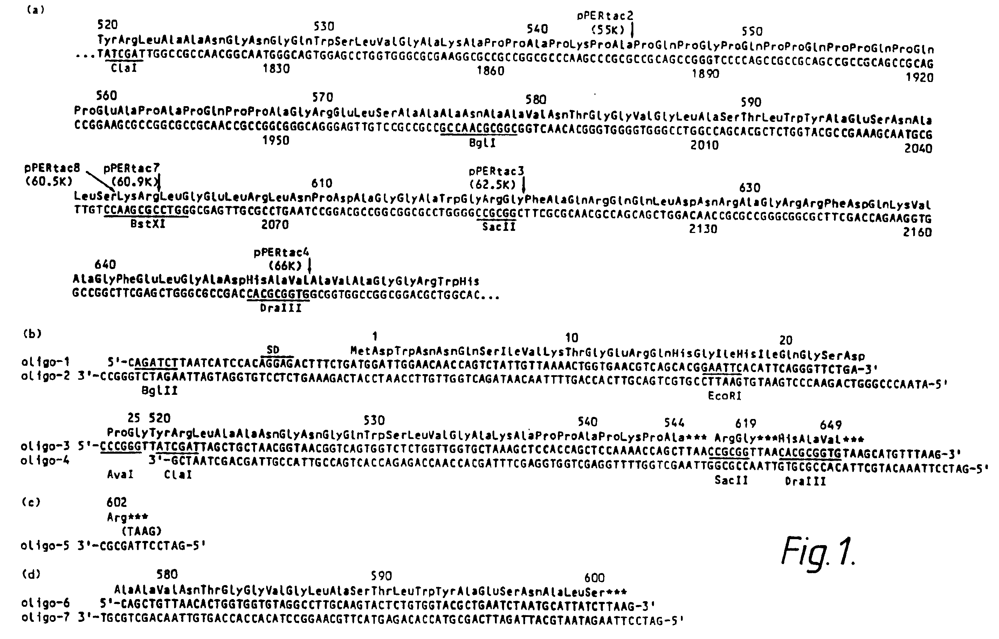

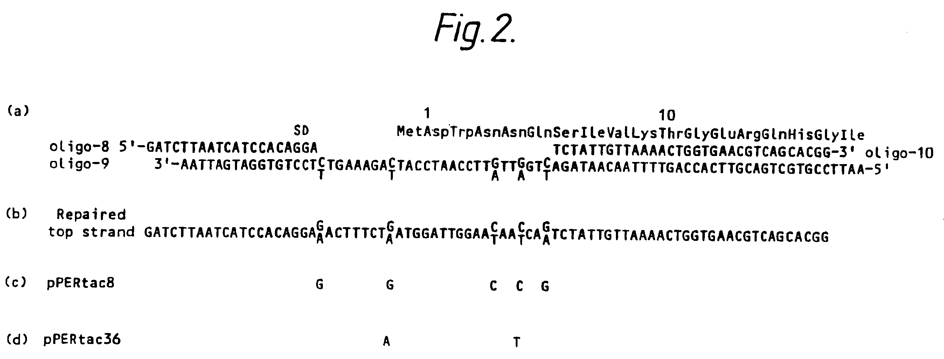

(i) Construction of plasmids: The intermediate plasmid pPERtac1 was constructed by cloning between the ApaI and BamHI sites of pTETtac2 (Makoff et al, Bio/Technology, 1989, 7, 1043-1046), the four oligonucleotides shown in Figure 1b (oligos 1-4). Plasmids pPERtac2, 3, 4, 7 and 8 (Figure 1a) were all constructed by cloning various B.pertussis P93 coding sequence fragments into pPERtac1. The fragments encoding P93 were obtained from pMLU6, a subclone of pBPI60 (Charles et al, P.N.A.S., 1989, 86, 3554-3558), using the following restriction sites: AvaI (315), Sall (1065) SphI (1250), ClaI (1806), BglI (1979), BstXI (2053), SacII (2101), DraIII (2190). [The numbers in brackets refer to the nucleotide numbers in the published sequence of P93 (Charles et al, P.N.A.S., 1989, 86, 3554-3558), part of which, from 1804 to 2217, is reproduced in Figure 1a]. Plasmid pPERtac2 was made by cloning into pPERtac1, an AvaI-ClaI 1491bp fragment from pMLU6, while plasmid pPERtac4 was made by cloning an AvaI-DraIII 1875bp fragment. Plasmid pPERtac3 was made by cloning a SphI-SacII 851bp fragment from pPERtac4 into pPERtac2. Plasmid pPERtac7 was constructed by cloning the single stranded oligonucleotide (oligo-5, Figure 1c) between the BstXI and BamHI sites of pPERtac4. Plasmid pPERtac8 was constructed by cloning the pair of oligonucleotides shown in Figure 1d (oligos 6 and 7) between the BglI and BamHI sites of pPERtac7. Comparison of the mobilities with native P.69 of the expression products of plasmids pPERtac 2, 3, 4, 7 and 8 plus the higher expressing two cistron derivatives (Makoff & Smallwood, 1990) are consistant with a C-terminus of P.69 at Ser600. (Makoff et al 1990 Nucl. Acids Res. 18, 1711-1718. In order to increase the expression of P.69 (terminating at Ser600), plasmid pPERtac36 was isolated from a number of transformants, obtained after annealing the mixed oligonucleotide oligo-9 to oligos 8 and 10 (Figure 2) and cloning between the BglII and EcoRI sites of pPERtac8, both of which originated in oligos 1 and 2 (Figure 1b). All oligonucleotides were synthesized, purified, kinased and the sequences confirmed as described previously (Makoff et al, Bio/Technology, 1989, 7, 1043-1046). E.coli strain MM294 (Meselson et al, Nature, 1968, 217, 1110-1114) was used throughout.
(ii) Induction and analysis of expressed protein: The expression plasmid, pPERtac36, contains a functional copy of the lacI gene and can therefore be induced by isopropyl-β-D-thiogalactopyranoside (IPTG). Cultures of E.coli MM294 containing pPERtac36 were grown overnight in L-broth (Maniatis et al, Molecular Cloning: A (Laboratory Manual, 1982, Cold Spring Harbor Laboratory) and diluted 1 in 5 into fresh medium containing 75µg/ml IPTG. Cells were grown for a further four hours with rapid aeration and harvested by centrifugation (10,000 x g, 10min). The 69kDa antigen was detected by Western blotting using polyclonal antisera raised in rabbits against the purified antigen. Levels of expression were quantified by densitometer scanning of SDS-polyacrylamide gels using a Joyce-Loebl Chromoscan 3 (Makoff et al, Nucl.Acids Res., 1989, 17, 10191-10202). This was determined for pPERtac36 as 36.0 ± 4.8% (Makoff et al Bio/Technology, 1990, 8, 1030-1033).
(iii) Refolding and purification: After centrifugation, the cell pellet was resuspended in a buffer comprising 50mM Tris-HCl, pH7.5, 50mM NaCl and lysed by two passages through a French Pressure cell (15,000 lb in ⁻²). The pellet fraction of the lysate was washed in buffer (A) (50mM Tris-HCl, pH8.0, 100mM NaCl, 1mM EDTA) comtaining 1% Triton X-100. The insoluble material (mostly comprising insoluble 69kDa antigen as inclusion bodies) was recovered by centrifugation and solubilized by resuspension in buffer (A) containing 6M guanidinium hydrochloride (GuHCl). The protein solution was adjusted to 6mg/ml and the GuHCl concentration reduced to 1M by dilution. The 69kDa antigen solution was placed in a dialysis bag and dialysed at 4^{o}C for 24 hours with 3 changes against buffer (A) so as to refold the protein. (Reviewed in Marston, Biochem.J., 1986, 2405, 1-12). The insoluble material that appears on dialysis was removed by centrifugation at 10,000 x g for 10 minutes.

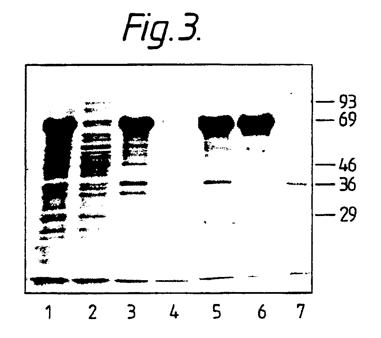

   Figure 3, shows an SDS-polyacrylamide gel (10%) of extracts of an induced culture of pPERtac36, during the solubilisation procedure. Lane 1, whole-cell extract; lane 2, first supernatant fraction; lane 3, first pellet fraction; lane 4, Triton X-100 wash supernatant fraction; lane 5, total fraction after solubilisation in GuHCl and its removal by dialysis; lane 6 supernatant fraction after dialysis; lane 7, pellet fraction after dialysis. Size markers are given in kDa.
   Soluble 69kDa antigen preparations were purified further by ion-exchange chromatography on Q-Sepharose. Samples were loaded on the column in 50mM Tris-HCl, pH8.0, washed and the antigen eluted with a 0-0.4M NaCl gradient. This purified material had below 0.1 endotoxin units per µg of protein by the Limulus amoebocyte lysate assay.

### Production in P.pastoris

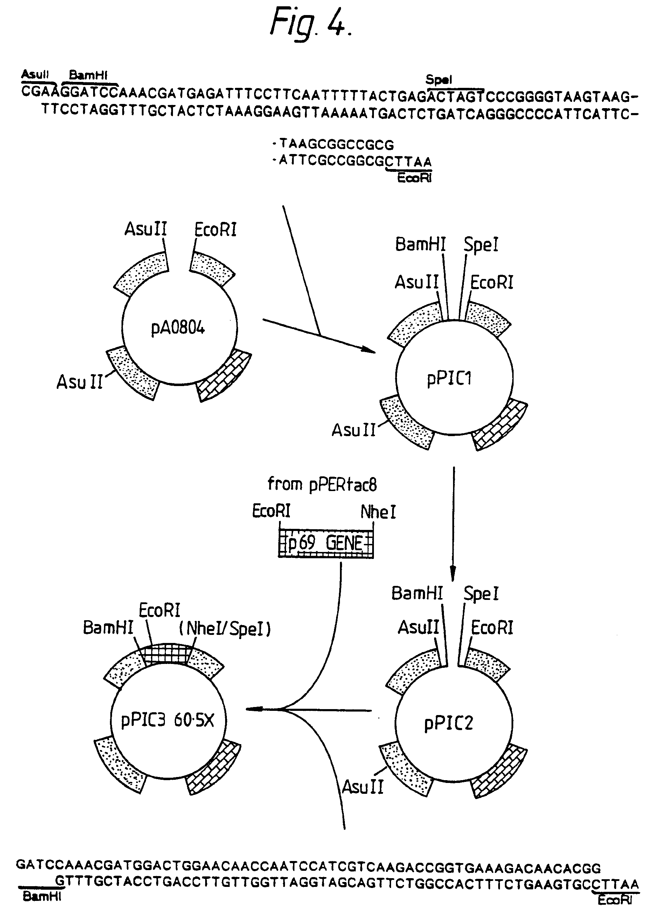

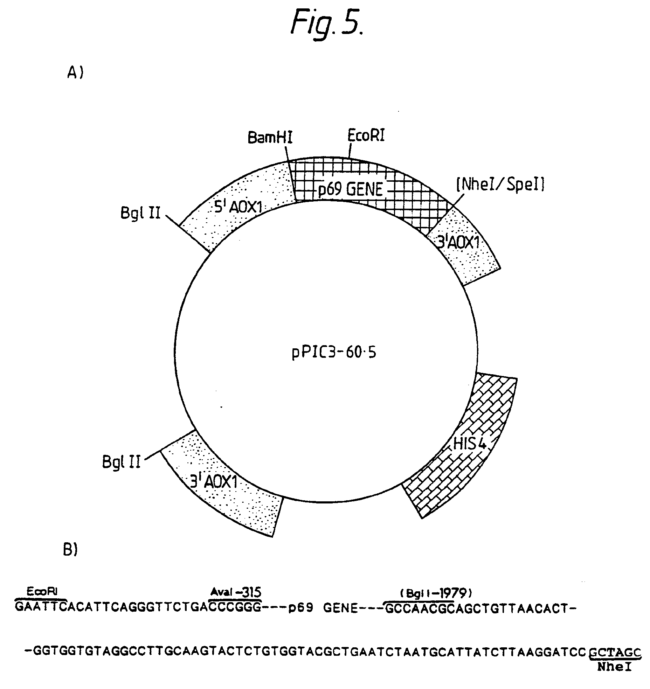

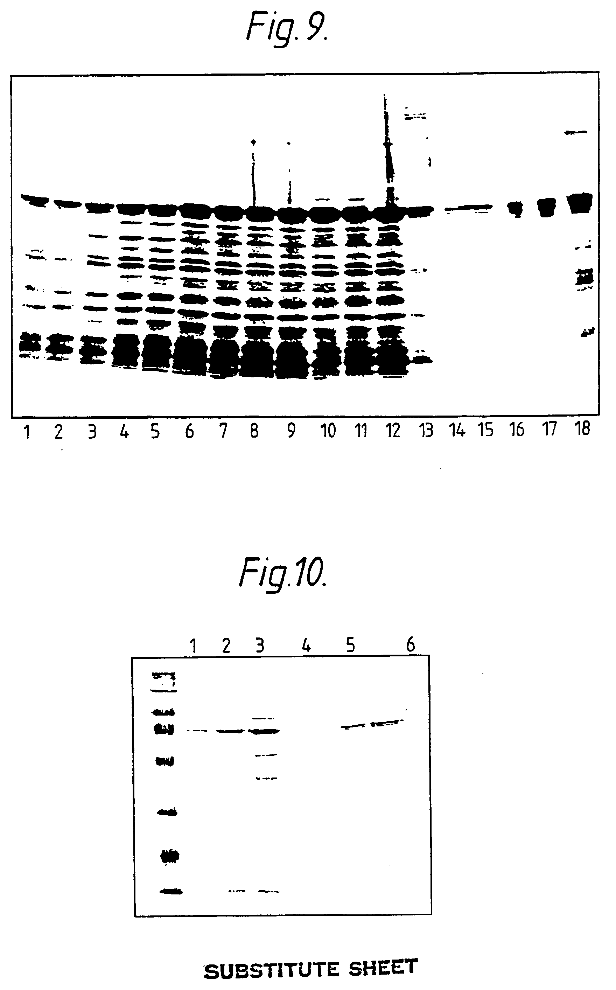

(i) Construction of expression vector: The vector pPIC3-60.5K, deposited at THE NATIONAL COLLECTION OF INDUSTRIAL AND MARINE BACTERIA LTD., P.O. Box 31, 135, Abbey Road, Aberdeen AB98DG, Scotland, UK on 30th March, 1990 under Accession No. 40270 and derived from pAO804 (K. Sreekrishna et al., Biochemistry, 1989, 28, 4117-4125), was used for intracellular expression of 69kDa antigen in Pichia pastoris. This vector uses the promoter from the AOX1 gene to drive expression and can be integrated into the host chromosomal AOX1 locus. To facilitate insertion of the gene encoding the 69kDa antigen, the synthetic oligonucleotides shown in Fig. 4 were cloned between the AsuII and EcoRI sites of pAO804 to give pPIC1. A derivative of this plasmid, pPIC2, which lacks the EcoRI site was then constructed. This was done by digesting with EcoRI followed by filling in of the protruding single stranded ends with the Klenow fragment of DNA polymerase I and ligating together the blunt ends. A 1.8kb EcoRI-NheI fragment (see Fig. 5) from the plasmid pPERtac8 which contains most of the gene encoding the 69kDa antigen was inserted into BamHI-SpeI cut pPIC2, using the adapter oligonucleotides shown in Fig. 4, to give pPIC3-60.5K. These oligonucleotides encode the 5' end of the gene including the initiator ATG codon.
(ii) Transformation: Pichia pastoris strain GS115 (his4⁻) was transformed with pPIC3-60.5K using the sphaeroplast method described by Cregg et al., Bio/Technology, 1987, 5, 479-485. Transformants were regenerated on minimal medium lacking histidine, so that His⁺ colonies would be selected. The transforming DNA was 10 or 20µg of BglII-digested pPIC3-60.5K. This digest contains two DNA fragments, one of which (7.2 kb) has AOX1 sequences at either end, so that it is targetted to integrate at and replace ('transplace') the chromosomal AOX1 gene.
   The His⁺ transformants generated are mainly found to contain undisrupted AOX1, and transplacements (aox1) may be isolated using a further screening procedure. Transplacements may be identified by their slow growth on methanol (Mut^{s} as opposed to Mut⁺ phenotype). The transformants can be picked directly off regeneration plates and tested for growth on minimal methanol plates (YNBBM agar). Alternatively, the regeneration top agars can be lifted and homogenised in water, and the yeast cells plated to about 300 colonies per plate on minimal glucose plates (YNBBD agar). Mut^{s} colonies are then identified by replica-plating onto minimal methanol plates. In general, the proportion of Mut^{s} is found to be 10-20% of all the transformants. Occasionally Mut⁺ transformants might be scored as Mut^{s}, and these may also prove to contain multiple copies of the vector.
(iii) Screening: In order to screen large numbers of transformants rapidly, they were grown in individual wells in a 96-place sterile microtitre plate (NUNCLON). 200µl of YPD broth in each well was inoculated with a Mut^{s} transformant, and the plates incubated for two days at 30^{o}C without shaking, to ensure growth to stationary phase. Included on each microtitre plate was a well containing GS115 (negative control) and one containing a known pPIC3-60.5K integrant (positive control). Using a multi-channel pipetter (Titertek), 50µl samples of each micro-culture were transferred onto a nitrocellulose filter on a Schleicher & Schuell 'minifold' under vacuum. The filters were air dried, marked for orientation, then treated in the following way to lyse the cells: (i) 15min at room temperature with 50mM EDTA, 2.5% 2-mercaptoethanol pH9.0, (ii) 4hrs at 37^{o}C with 1mg/ml zymolyase (100T) in water, (iii) 5min at room temperature in 0.1M NaOH, 1.5M NaCl end (iv) twice for 5min at room temperature in 2 x SSC. Each treatment was performed by soaking 2 sheets of 3MM paper with the solution and placing the nitrocellulose filter on top. After these treatments the filters were baked at 80^{o}C for 1hr.
   The filters were probed by Southern hybridization using 69kDa antigen-specific DNA radiolabelled to high specific activity using random-primed labelling (Feinberg et al, Anal. Biochem., 1989, 132, 6-13). Standard methods were used for prehybridization, hybridization, washing and autoradiography.

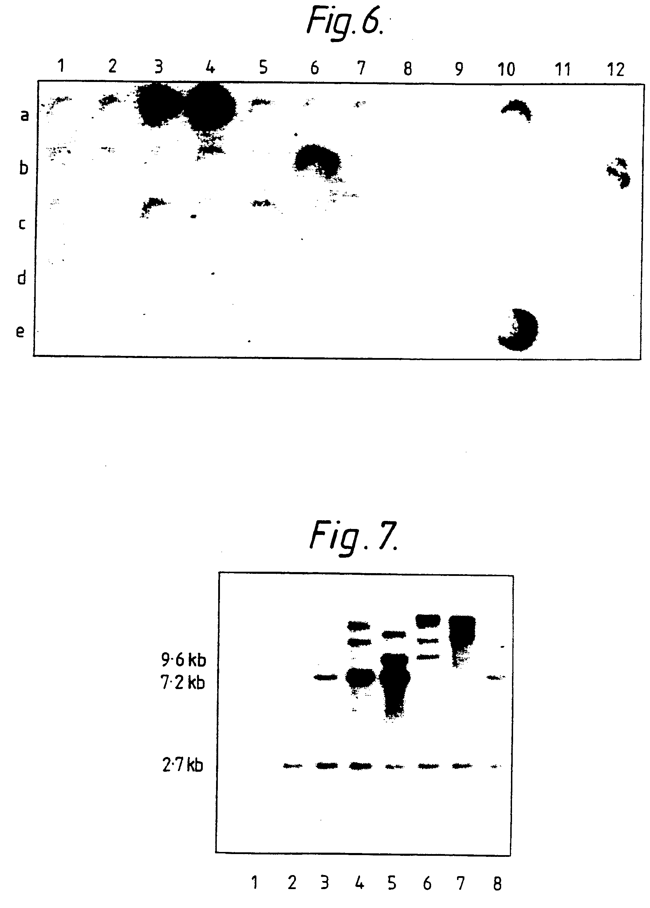

   Fig. 6 shows the results of such a screen of over 200 Mut^{s} transformants of pPIC3-60.5K. All the transformants reacted with the probe and most gave a similar weak signal (single-copy transformants). However, a very small proportion gave much stronger signals for Example A3 and 4. These multi-copy transformants were tested further. The positive control in the filter shown is a transformant previously identified as multi-copy.
   The DNA from two selected single-copy transplacements and four multi-copy transformants identified by dot blots was further analysed by Southern blotting. DNA was prepared from 50ml cultures of Pichia cells according to Sherman, F.,et al., (Methods in Yeast Genetics, 1983, Cold Spring Harbor, New York). BglII digests were separated in a 0.6% agarose gel, transferred to nitrocellulose by Southern blotting, and hybridised with radio-labelled HIS4 DNA (Fig. 7). With the BglII-cut vector, pPIC3-60.5K (track 1), a 7.2 kb band corresponding to the expression cassette was observed, whereas the untransformed strain GS115 gave the chromosomal 2.7 kb HIS4 fragment (track 2). Single transformants gave both these bands (tracks 3 and 8), while two of the multi-copy transformants had an increased intensity of the 7.2 kb band, indicating multiple insertion of the expression cassette (tracks 4 and 5). The other two multi-copy transformants did not give a 7.2 kb band et all, but showed higher molecular weight bands which are multimeric forms lacking BglII sites.
(iv) Protein Analysis: Selected transformants were cultured in YNBBGCas for 2 days at 30^{o}C to reach stationary phase. These starter cultures were diluted to an OD₆₀₀ of 0.25 in 5ml fresh YNBBGCas and grown for 6hr. To induce these cultures the cells were collected by centrifugation, washed once in sterile water, and resuspended in YNBBMCas. Inductions were carried out for 2 days at 30^{o}C.
   Cells were then harvested by low speed centrifugation, washed once in water and suspended 0.5ml in ice cold break buffer (20mM sodium phosphate pH7.0, 0.1% triton X-100, 4mM phenylmethyl sulphonylfluoride). Acid washed glass beads (0.45mm) were added and the cells were broken by vigorous vortexing. The protein concentration of the extracts was determined using the BioRad protein assay (BioRad, according to manufacturer's instructions) and the material was stored at -20^{o}C.
   Proteins were separated by electrophoresis in 7.5% SDS-polyacrylamide gels (Laemmli U.K., Nature, 1970, 227: 680-785). The proteins were visualised in the gel staining with Coomassie Brilliant Blue R. Alternatively the proteins were transferred to a nitrocellulose filter and reacted with the 69kDa antigen specific monoclonal antibody BB05 (Montaraz et al., Infect. Immunity, 1985, 47, 744-751); deposited at PHLS Public Health Laboratory Service Centre for Applied Microbiology and Research, Porton Down, Salisbury, Wiltshire, U.K. in the European collection of Animal cell cultures under Accession No. 90020103, on the 1st February, 1990 in accordance with the terms of the Budapest treaty), then with goat anti-mouse IgG conjugated to horse-radish peroxidase, and developed with H₂0₂ and 4-chloronaphthol (BioRad). In this way. the expressed 69kDa antigen could be specifically detected.

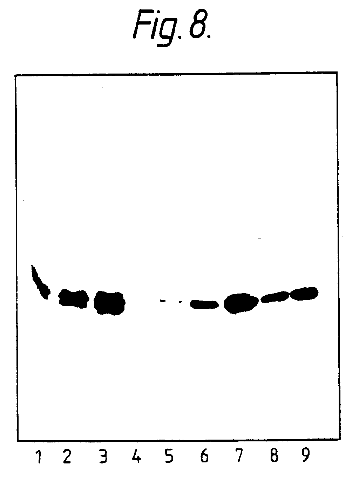

   Fig. 8 shows a Western blot of induced extracts of 4 selected multi-copy transformants (clone nos. 3, 4, 18 and 22) and one typical single-copy transformant. By comparison to standard concentrations of pure 69kDa antigen, it can be estimated that single-copy transplacements express 69kDa antigen at 0.1-0.5% of total cell proteins (t.c.p.), clone no. 22 at approx. 2%. and clone 4 at approx. 5%. These levels are for sub-optimal induction conditions. In fermenter inductions a 5-fold improvement was seen for clone 22, to 10% t.c.p,. However, clone 4, which turned out to be Mut⁺, remained at 6% t.c.p. in the fermenter. In low-expressing transformants the 69kDa antigen is largely soluble, whereas at high expression levels it is mainly (90%) insoluble. This is preferable since the material can be readily isolated, renatured and purified from the insoluble fraction.
(v) Fermentation : Production of 69kDa antigen by high cell density Pichia pastoris cultures was carried out using clone 22 in a 2L Braun fermenter equipped with monitors and controls for pH, dissolved O₂, stirring speed, temperature and air flow. A 10ml YNBBG overnight culture was used to inoculate the fermenter containing 1 litre of 5 x basal salts (phosphoric acid, 42mls/L; calcium sulphate 2H₂O, 1.8g/L; potassium sulphate 28.6g/L; magnesium sulphate 7H₂O, 23.4g/L: potassium hydroxide, 6.5g/L) with 4ml of PTM₁ salts (cupric sulphate 5H₂O, 6g/L; potassium iodide, 0.08g/L; manganese sulphate H₂O, 3g/L; sodium molybdate, 0.2g/L: ferrous sulphate 7H₂O, 65h/L; biotin, 0.2g/L; sulphuric acid, 5ml/L) and 5% (v/v) glycerol at 30^{o}C. Dissolved oxygen was maintained above 20% by adjusting aeration and agitation, and the pH was maintained at pH5.0 by the addition of 50% (v/v) ammonium hydroxide. Growth was continued until the glycerol was exhausted (24-30hr). A limited glycerol feed (containing 50% w/v glycerol and 12ml/L PTM₁ salts) was then initiated at 12ml/hr for 17-21 hr. After this period the culture was induced by replacing the glycerol feed with a methanol feed (100% methanol plus 12ml/L PTM₁ salts) at 1ml/hr for 2hr. Then the methanol feed rate was gradually increased over a period of 6 hr to 6ml/hr and the fermentation was continued using these conditions for a further 40hr. At this point the methanol feed rate was reduced to 2ml/hr.
   Samples were taken from the fermenter at different times after induction and were analysed for 69kDa antigen expression as described above. The results of a Western blot analysis are shown in Fig 9, indicating that levels of the 69kDa antigen of about 10% of cell protein were achieved.
(vi) Renaturation: Fermenter samples from 28hr and 32hr after the start of induction, containing 30ml of cells at OD₆₀₀ of 220, were pooled for processing. The cells were harvested by low speed centrifugation, washed once in water, then taken up to 30ml with buffer A (50mM Tris pH8.0, 0.1M NaCl, 1mM EDTA) containing 1% Triton X-100. The cells were then broken in a bead-beater (Biospec Products, Bartlesville, O.K.) with 0.45mm glass beads in ten one-minute pulses. The cell lysate was cleared by centrifugation at 15,000rpm for 30min (Sorvall, RC-5B, SS-34 rotor). Pellets were washed by suspending in buffer A and re-centrifuging. The pellet of insoluble protein was resuspended in buffer A (6ml) and 6 volumes of 7.0M guanidinium chloride, 50mM Tris pH8.0, 1mM EDTA were added to solubilise proteins. This solution was diluted further with 7M guanidinium chloride to 110ml and dialysed extensively against 5L of buffer A at 4^{o}C. The material in the dialysis bag was then cleared by centrifugation (40min, 10,000rpm, RC-5B, SS-34 rotor). The final supernatant, containing solubilised 69kDa antigen, was concentrated by dialysing against solid Sephadex G-100.
   Equivalent amounts of fractions from each stage of this procedure were analysed by SDS-PACE followed by Western blotting. The results indicate that about 50% of the 69kDa antigen was solubilised in this particular experiment but that all of this remained soluble after dialysis. A Coomassie-stained gel indicated that the purity of the final material was high (60.70%; Fig. 10).

### Example 2: Preparation and Inactivation of Toxoided LPF

Cultures of B.pertussis can be grown in flasks or in fermentors in a modified Stainer-Scholte medium supplemented with heptakis-(2.6-0-dimethyl)-β-cyclodextrin (MeBCD) (Imaizumi et al., Infect. Immun., 1983, 41: 1138-1143. LPF can be purified from the culture supernatant by column or batch adsorption on hydroxylapatite agarose (Sato et al., "Seminars in Infectious Disease, 1982, vol IV" 380-5, Threme-Stratton, Inc. New York). The hydroxylapatite is first equilibrated with O.O1M phosphate buffer pH8.0. The culture supernatant can then be applied to the column and subsequently washed with a solution of 0.01M phosphate buffer pH6.0. Elution of LPF is carried out using 0.1M phosphate buffer pH7.O containing 0.5m NaCl. The LPF may be further purified, for example, on a haptoglobin-Sepharose 4B column (Imaizumi et al., Infect & Immun, 1983, 41, 1138-1143).

Inactivation or 'toxoiding' of purified preparations of LPF may be carried out using a variety of different inactivating agents, such as formaldehyde, glutaraldehyde hydrogen peroxide or formalin (Sato et al, Lancet, 1984, (1), 122-126), or a combination of any of these reagents.

### Example 3: Biological Potency in the Kendrick Test

The test was performed according to W.H.O. requirements for Pertussis Vaccine using outbred NIH-S mice (OLAC, category 3, free of most pathogens including B.bronchiseptica), weighing 14-16g. The antigens, in 0.5 ml volumes, wore inoculated intraperitoneally as a mixture, and comprised a top dilution and three three-fold serial dilutions. After two weeks the mice were challenged intracerebrally using the recommended challenge strain 18-323 (-400 LD₅₀). The number of survivors in each group was used for calculation of the relative potency in respect of the British Pertussis Reference Vaccine 66/84 (J.Biological Standard, 1981, 9, 351-365) using a program of parallel line probit analysis. The results are shown in Tables 1, 2 and 3. Examination of these data shows limited protection for either the 69kDa antigen or toxoided LPF when used alone as vaccines. In contrast, the combination of the 69kDa antigen and toxoided LPF exhibits a surprising synergy giving rise to levels of protection very similar to the whole cell reference vaccine.

**Table 1**

| No. | Name and Description | | Survivors | | |
|---|---|---|---|---|---|
| | | Dose | Male | Female | Total |
| 1 | 69kDa (E.coli) 80µg | neat | 1/9 | 0/9 | 1/18 |
| | | 1/4 | 0/9 | 2/9 | 2/18 |
| | | 1/16 | 0/9 | 0/9 | 0/18 |
| | | 1/32 | 0/9 | 0/9 | 0/18 |
| 2 | Ref vaccine | neat | 7/9 | 6/9 | 13/18 |
| | | 1/4 | 4/9 | 4/9 | 8/18 |
| | | 1/16 | 3/9 | 2/9 | 5/18 |
| | | 1/32 | 0/9 | 0/9 | 0/18 |
| 3 | Challenge | neat | 0/9 | 0/9 | 0/18 |
| | | 1/50 | 0/9 | 0/9 | 0/18 |
| | | 1/250 | 1/9 | 1/9 | 2/18 |
| | | 1/1250 | 2/9 | 4/9 | 6/18 |

**Table 2**

| No. | Name and Description | | Survivors | | |
|---|---|---|---|---|---|
| | | Dose | Male | Female | Total |
| 1 | Toxoid 5µg LPF | neat | 3/9 | 7/9 | 10/18 |
| | | 1/4 | 0/9 | 2/9 | 2/18 |
| | | 1/16 | 0/9 | 0/9 | 0/18 |
| | | 1/64 | 0/9 | 1/9 | 1/18 |
| 2 | Toxoid 5µg LPF 69kDa (E.coli) 20µg | neat | 9/9 | 8/9 | 17/18 |
| | | 1/4 | 3/9 | 5/9 | 8/18 |
| | | 1/16 | 1/9 | 0/9 | 1/18 |
| | | 1/64 | 0/9 | 0/9 | 0/18 |
| 3 | Ref vaccine | neat | 6/9 | 9/9 | 15/18 |
| | | 1/4 | 2/9 | 5/9 | 7/18 |
| | | 1/16 | 0/9 | 1/9 | 1/18 |
| | | 1/64 | 0/9 | 1/9 | 1/18 |
| 4 | Challenge | neat | 0/5 | 0/5 | 0/10 |
| | | 1/50 | 0/5 | 0/5 | 0/10 |
| | | 1/250 | 0/5 | 1/5 | 1/10 |
| | | 1/1250 | 3/5 | 4/5 | 7/10 |

**Table 3**

| No | Name and Description | | Survivors | | |
|---|---|---|---|---|---|
| | | Dose | Male | Female | Total |
| 1 | Toxoid 20µg LPF | neat | 4/9 | 4/9 | 8/18 |
| | | 1/4 | 1/9 | 2/8 | 3/17 |
| | | 1/16 | 1/9 | 0/9 | 1/18 |
| | | 1/64 | 2/9 | 0/9 | 2/18 |
| 2 | Toxoid 20µg LPF 69kDa 20µg(Pichia) | neat | 8/9 | 7/9 | 15/18 |
| | | 1/4 | 3/9 | 7/9 | 10/18 |
| | | 1/16 | 0/9 | 3/9 | 3/18 |
| | | 1/64 | 0/9 | 0/9 | 0/18 |
| 3 | Ref vaccine | neat | 7/9 | 8/9 | 15/18 |
| | | 1/4 | 2/9 | 5/9 | 7/18 |
| | | 1/16 | 1/9 | 1/9 | 2/18 |
| | | 1/64 | 0/9 | 1/9 | 1/18 |
| 4 | Challenge | neat | 0/5 | 0/5 | 0/10 |
| | | 1/50 | 1/5 | 0/5 | 1/10 |
| | | 1/250 | 2/5 | 1/5 | 3/10 |
| | | 1/1250 | 4/5 | 3/5 | 7/10 |

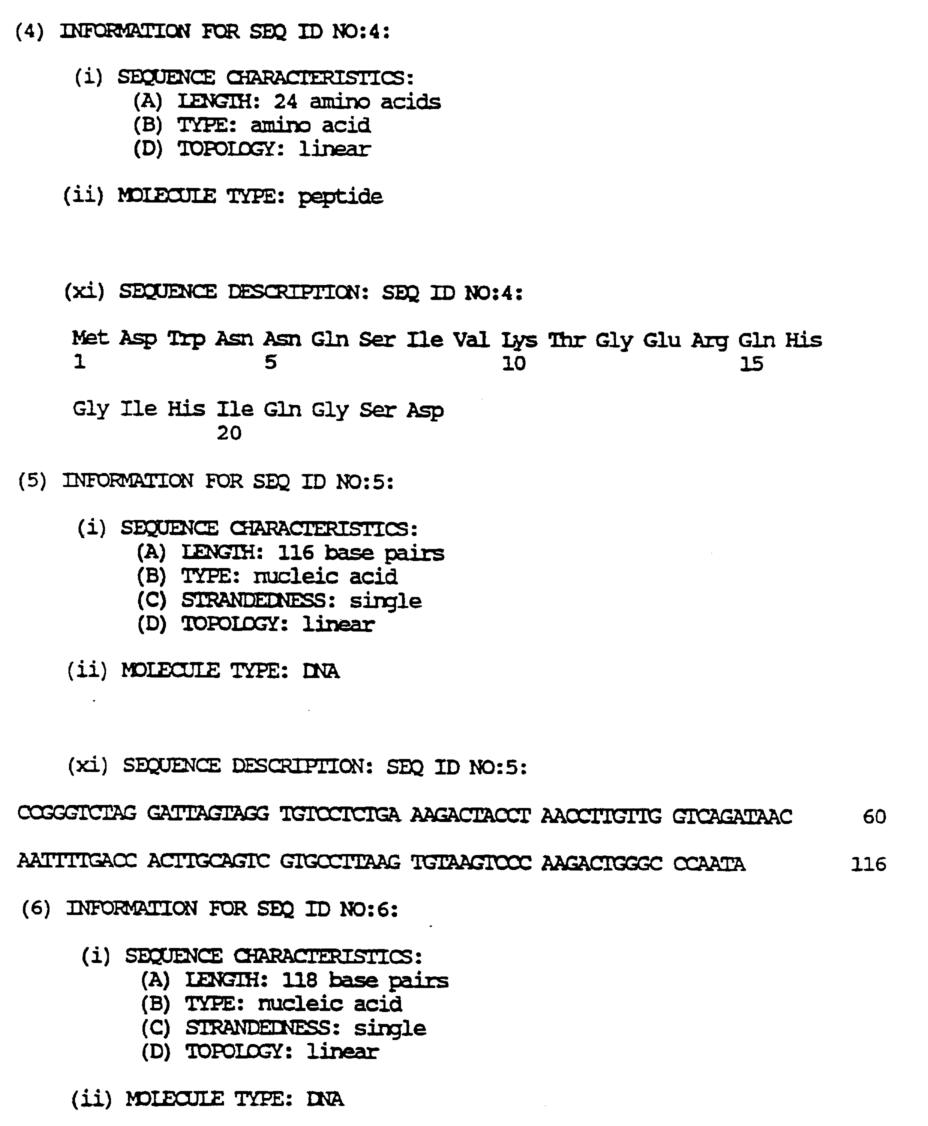

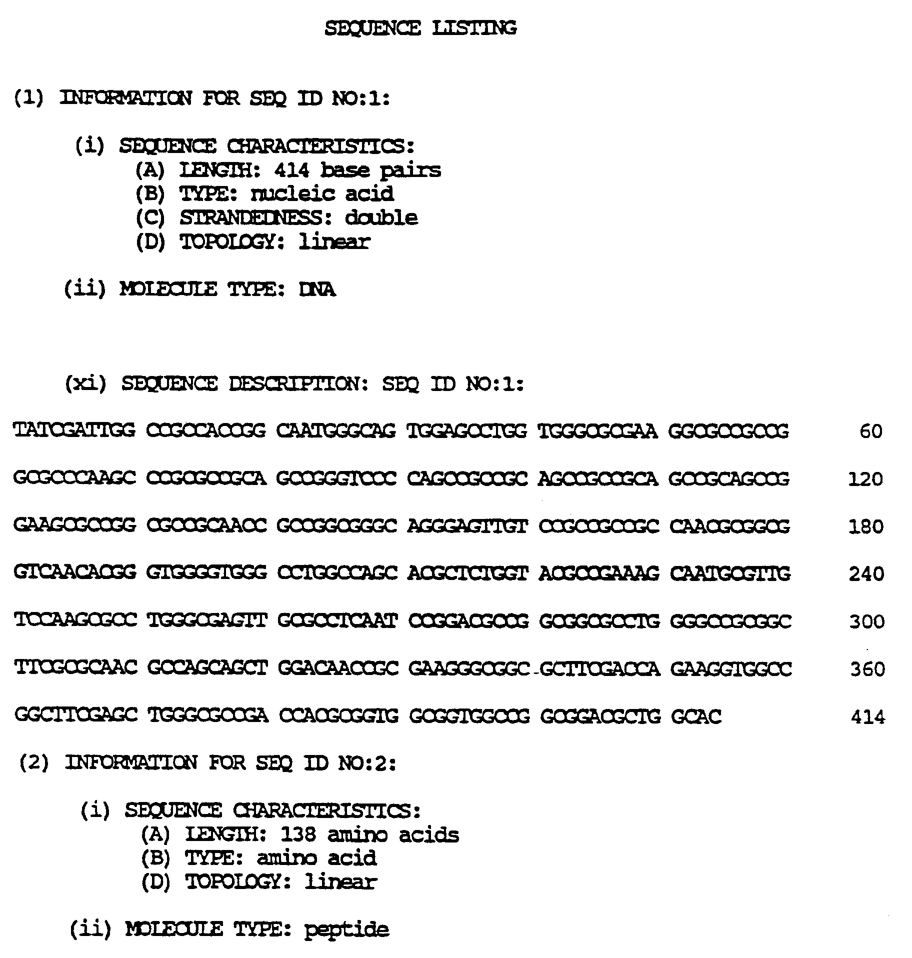

(xi) SEQUENCE DESCRIPTION: SEQ ID NO:2:

Tyr Arg Leu Ala Ala Asn Gly Asn Gly Gln Trp Ser Leu Val Gly Ala
1               5                   10                  15

Lys Ala Pro Pro Ala Pro Lys Pro Ala Pro Gln Pro Gly Pro Gln Pro
            20                  25                  30

Pro Gln Pro Pro Gln Pro Gln Pro Glu Ala Pro Ala Pro Gln Pro Pro
        35                  40                  45

Ala Gly Arg Glu Leu Ser Ala Ala Ala Asn Ala Ala Val Asn Thr Gly
    50                  55                  60

Gly Val Gly Leu Ala Ser Thr Leu Trp Tyr Ala Glu Ser Asn Ala Leu
65                  70                  75                  80

Ser Lys Arg Leu Gly Glu Leu Arg Leu Asn Pro Asp Ala Gly Gly Ala
                85                  90                  95

Trp Gly Arg Gly Phe Ala Gln Arg Gln Gln Leu Asp Asn Arg Ala Gly
            100                 105                 110

Arg Arg Phe Asp Gln Lys Val Ala Gln Phe Glu Leu Gly Ala Asp His
        115                 120                 125

Ala Val Ala Val Ala Gly Gly Arg Trp His
    130                 135

(3) INFORMATION FOR SEQ ID NO:3:

(i) SEQUENCE CHARACTERISTICS:
(A) LENGTH: 102 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:3:

CAGATCTTAA TCATCCACAG GAGACTTTCT GATGGATTGG AACAACCAGT CTATTGTTAA 60

AACTGGTGAA CGTCAGCACG GAATTCACAT GCAGGGTTCT GA 102

(xi) SEQUENCE DESCRIPTION: SEQ ID NO:6:

CCCGGGTTAT CGATTAGCTG CTAACGGTAA CGGTCAGTGG TCTCTGGTTG GTGCTAAAGC 60

TCCACCAGCT CCAAAACCAG CTTAACCGCG GTTAACACGC GGTGTAAGCA TGTTTAAG 118

(7) INFORMATION FOR SEQ ID NO:7:

(i) SEQUENCE CHARACTERISTICS:
(A) LENGTH: 27 amino acids
(B) TYPE: amino acid
(D) TOPOLOGY: linear

(ii) MOLECULE TYPE: peptide

(xi) SEQUENCE DESCRIPTION: SEQ ID NO:7:

Pro Gly Tyr Arg Leu Ala Ala Asn Gly Asn Gly Gln Trp Ser Leu Val
1               5                   10                  15

Gly Ala Lys Ala Pro Pro Ala Pro Lys Pro Ala
            20                  25

(8) INFORMATION FOR SEQ ID NO:8:

(i) SEQUENCE CHARACTERISTICS:
(A) LENGTH: 112 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:8:

GCTAATCGAC GATTGCCATT GCCAGTCACC AGAGACCAAC CAGCATTTCG AGGTGGTCGA 60

GGTTTTGGTC GAATTGGCGC CAATTGTGCG CCAGATTCGT ACAAATTCCT AG 112

(9) INFORMATION FOR SEQ ID NO:9:

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:9:

CGCGATTCCT AG 12

(10) INFORMATION FOR SEQ ID NO:10:

(i) SEQUENCE CHARACTERISTICS:
(A) LENGTH: 72 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:10:

CAGCTGTTAA CACTGGTGGT GTAGGCCTTG CAAGTACTCT GTGGTACGCT GAATCTAATG 60

CATTATCTTA AG 72

(11) INFORMATION FOR SEQ ID NO:11:

(i) SEQUENCE CHARACTERISTICS:
(A) LENGTH: 23 amino acids
(B) TYPE: amino acid
(D) TOPOLOGY: linear

(ii) MOLECULE TYPE: peptide

(xi) SEQUENCE DESCRIPTION: SEQ ID NO:11:

Ala Ala Val Asn Thr Gly Gly Val Gly Leu Ala Ser Thr Leu Trp Tyr
1               5                   10                  15

Ala Glu Ser Asn Ala Leu Ser
            20

(12) INFORMATION FOR SEQ ID NO:12:

(i) SEQUENCE CHARACTERISTICS:
(A) LENGTH: 79 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:12:

TGCGTCGACA ATTGTGACCA CCACATCCGG AACGTTCATG AGACACCATG CGACTTAGAT 60

TACGTAATAG AATTCCTAG 79

(13) INFORMATION FOR SEQ ID NO:13:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:13:

GATCTTAATC ATCCACAGGA 20

(14) INFORMATION FOR SEQ ID NO:14:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:14:

TCTATTGTTA AAACTGGTGA ACGTCAGCAC GG 32

(15) INFORMATION FOR SEQ ID NO:15:

(i) SEQUENCE CHARACTERISTICS:
(A) LENGTH: 18 amino acids
(B) TYPE: amino acid
(D) TOPOLOGY: linear

(ii) MOLECULE TYPE: peptide

(xi) SEQUENCE DESCRIPTION: SEQ ID NO:15:

Met Asp Trp Asn Asn Gln Ser Ile Val Lys Thr Gly Glu Arg Gln His
1               5                   10                  15

Gly Ile

(16) INFORMATION FOR SEQ ID NO:16:

(i) SEQUENCE CHARACTERISTICS:
(A) LENGTH: 79 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:16:

AATTAGTAGG TGTCCTYTGA AAGAYTACCT AACCTTRTTR GTYAGATAAC AATTTTGACC     60

ACTTGCAGTC GTGCCTTAA     79

(17) INFORMATION FOR SEQ ID NO:17:

(i) SEQUENCE CHARACTERISTICS:
(A) LENGTH: 79 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:17:

GATCTTAATC ATCCACAGGA RACTTTCTRA TGGATTGGAA YAAYCARTCT ATTGTTAAAA     60

CTGGTGAACG TCAGCACGG     79

(18) INFORMATION FOR SEQ ID NO:18:

(i) SEQUENCE CHARACTERISTICS:
(A) LENGTH: 75 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:18:

CGAAGGATCC AAACGATGAG ATTTCCTTCA ATTTTTACTG AGACTAGTCC CGGGGTAAGT 60

AAGTAAGCGG CCGCG 75

(19) INFORMATION FOR SEQ ID NO:19:

(i) SEQUENCE CHARACTERISTICS:
(A) LENGTH: 77 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:19:

TTCCTAGGTT TGCTACTCTA AAGGAAGTTA AAAATGACTC TGATCAGGGC CCCATTCATT 60

CATTCGCCGG CGCTTAA 77

(20) INFORMATION FOR SEQ ID NO:20:

(i) SEQUENCE CHARACTERISTICS:
(A) LENGTH: 60 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:20:

GATCCAAACG ATGGACTGGA ACAACCAATC CATCGTCAAG ACCGGTGAAA GACAACACGG 60

(21) INFORMATION FOR SEQ ID NO:21:

(i) SEQUENCE CHARACTERISTICS:
(A) LENGTH: 60 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:21:

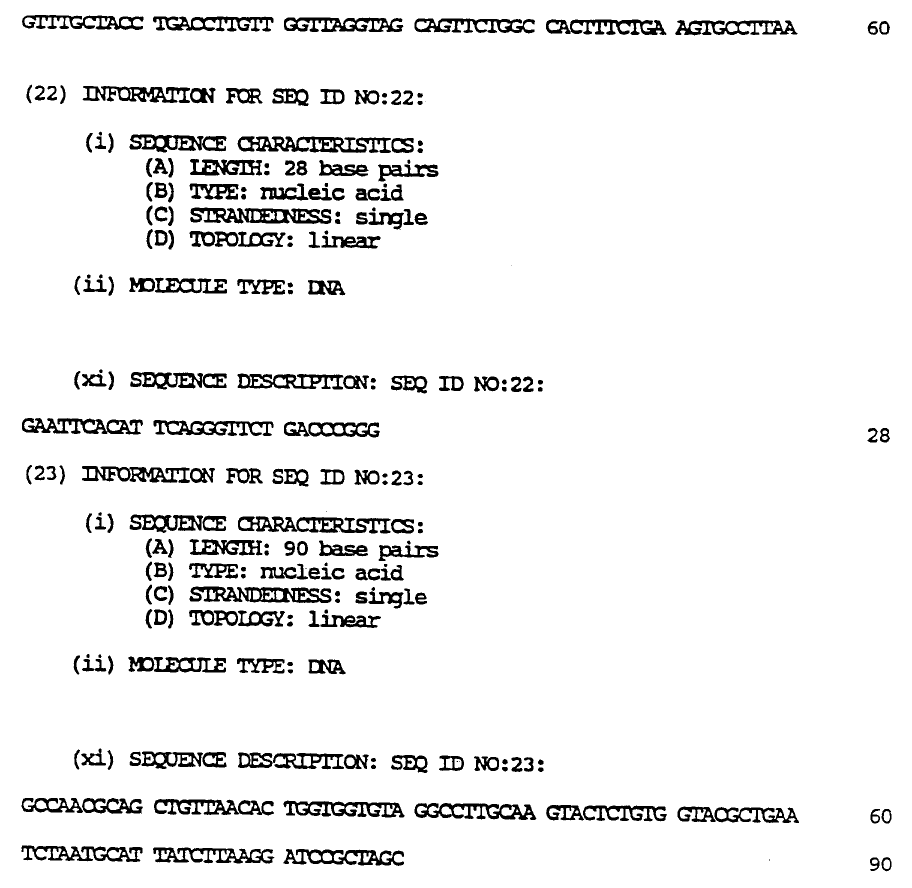

## Claims

1. A vaccine containing a synergistic combination of the 69kDa antigen of Bordetella pertussis and toxoided lymphocytosis promoting factor of Bordetella pertussis.

2. A vaccine according to claim 1, in which the 69kDa antigen has been obtained using recombinant DNA technology.

3. A vaccine according to claim 1 or 2, in which the lymphocytosis promoting factor has been toxoided using an inactivating agent selected from formaldehyde, glutaraldehyde, hydrogen peroxide or a combination thereof.

4. A vaccine according to any one of the preceding claims, in which the weight ratio of the 69kDa antigen to the toxoided lymphocytosis promoting factor is approximately 1:1.

5. A vaccine according to any one of the preceding claims, which further comprises a pharmaceutically acceptable liquid vehicle suitable for parenteral administration.

6. A vaccine according to claim 5, in which the concentration of antigenic protein is from 0.03 to 2 mg/ml.

7. A vaccine according to any one of the preceding claims, which further comprises an adjuvant.

8. A vaccine according to any one of the preceding claims, which further comprises another antigen of Bordetella pertussis or an antigen of diphtheria or tetanus.

9. A process for the preparation of a vaccine as claimed in claim 1, which process comprises mixing the 69kDa antigen of Bordetella pertussis and toxoided lymphocytosis promoting factor of Bordetella pertussis in such amounts as to provide a synergistic combination thereof.

10. A method for inducing immunity to whooping cough in a human patient, which method comprises administering thereto an effective amount of a vaccine as claimed in any one of claims 1 to 8.
